(19) Europäisches Patentamt
European Patent Office
Office européen des brevets

(11) **EP 4 506 688 A1**

(12) **EUROPEAN PATENT APPLICATION**

(43) Date of publication:
**12.02.2025 Bulletin 2025/07**

(21) Application number: **24193702.8**

(22) Date of filing: **08.08.2024**

(51) International Patent Classification (IPC):
**G01N 33/00** (2006.01) **A01G 9/26** (2006.01)

(52) Cooperative Patent Classification (CPC):
**G01N 33/0098; A01G 7/00; A01G 9/26**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.08.2023 GB 202312301**

(71) Applicant: **Vivent SA**
**1196 Gland (CH)**

(72) Inventors:
• **Kurenda, Andrzej**
**1196 Gland (CH)**

• **Carroll, Caleb**
**1196 Gland (CH)**
• **Wallbridge, Nigel Christopher**
**1196 Gland (CH)**
• **Barker, Nicholas**
**1196 Gland (CH)**
• **Plummer, Carrol Annette**
**1196 Gland (CH)**
• **Knulst, Hubert**
**1196 Gland (CH)**
• **Lecci, Sandro**
**1196 Gland (CH)**

(74) Representative: **Marks & Clerk LLP**
**15 Fetter Lane**
**London EC4A 1BW (GB)**

(54) **SYSTEM AND METHOD FOR ASSESSING A HEALTH STATE OF A GROUP OF PLANTS WITHIN A GROWTH ENVIRONMENT**

(57) There is provided a method of assessing a health state of a group of plants within a growth environment 50. The method comprises: for each of a plurality of plant specimens 2, 2' within the growth environment 50 with the plurality of plant specimens being a subset of the group of plants: obtaining an electrical signal 15 from a respective plant specimen during a first time period; comparing the obtained electrical signal with data 18, 20 indicative of a historical electrical signal, with the historical electrical signal obtained from the respective plant specimen during a second time period; and generating plant data 22, 24, 26 based upon a comparison of the obtained electrical signal with the data indicative of the historical electrical signal, wherein the plant data is indicative of an extent of divergence between the obtained electrical signal and the historical electrical signal for the respective plant specimen; assessing the health state of the group of plants within the growth environment during the first time period, based upon the plant data of the plurality of plant specimens.

Figure 4

**Description**

Technical Field

The present disclosure relates to a method and a system for assessing a health state of a group of plants within a growth environment. More particularly, but not exclusively, the method and the system assess the health state of the group of plants, by comparing current and historical electrical signals obtained from a plurality of plant specimens which are a subset of the group of plants.

Background

It is known that stable circadian (nycthemeral) rhythms are linked to positive health states and that unstable or disrupted rhythms are linked to unhealthy states. These rhythms can be measured in a variety of ways including the measurement of electrical potential differences across cell membranes. Furthermore, it is known that electrical potential differences, or membrane potentials, are maintained across cell membranes in plants, and that electrical potential differences across cell membranes in plants are influenced by a range of environmental changes such as light levels, temperature, humidity, soil moisture and conductivity and the presence of diseases and pests.

However, it remains as a challenge to efficiently detect a health status of a group of plants with a reasonable-degree of confidence based upon electrical signals obtained from the plants.

Thus, there is a need to provide an improved method and system for accessing a health state of a group of plants within a growth environment.

Summary

According to a first aspect of the present disclosure, there is provided method of assessing a health state of a group of plants within a growth environment, comprising: for each of a plurality of plant specimens within the growth environment, the plurality of plant specimens being a subset of the group of plants: obtaining an electrical signal from a respective plant specimen during a first time period; comparing the obtained electrical signal with data indicative of a historical electrical signal, with the historical electrical signal obtained from the respective plant specimen during a second time period; and generating plant data based upon a comparison of the obtained electrical signal with the data indicative of the historical electrical signal, wherein the plant data is indicative of an extent of divergence between the obtained electrical signal and the historical electrical signal for the respective plant specimen; and assessing the health state of the group of plants within the growth environment during the first time period, based upon the plant data of the plurality of plant specimens.

Advantageously, the first aspect of the present disclosure provides a method for efficiently assessing an overall health status of plants within a growth environment, irrespective of the type of the plants or the growing conditions of the growth environment. In particular, the plurality of plant specimens act as sentinel plants to represent the overall health state of the group of plants, and the health state of the group is assessed purely based upon a comparison of recent and historical electrical signals obtained from the plant specimens. The method of the first aspect does not require any reference information or user input relating to plant genetics, climate conditions, and/or particulars of the growth environment. Further, the method of the first aspect may be performed during the first time period, thereby detecting the health state of the group of plants in near real-time.

The growth environment may comprise a controlled growth environment (such as a glasshouse, a greenhouse, a polytunnel, an indoor farm, or a vertical farm), or an open field.

The group of plants may be of the same type.

The plurality of plant specimens may comprise at least four plant specimens scattered within the growth environment.

The data indicative of the historical electrical signal may represent a range of an amplitude of the historical electrical signal.

Comparing the obtained electrical signal with data indicative of the historical electrical signal may comprise comparing an amplitude of the obtained electrical signal with the range of the amplitude of the historical electrical signal.

Generating the plant data may comprise generating first plant specimen data indicating a percentage of time when the amplitude of the obtained electrical signal stays within the range, and generating second plant specimen data indicating an amount of deviation between the amplitude of the obtained electrical signal and the range.

In other words, the plant data comprises the first plant specimen data and the second plant specimen data. The second plant specimen data may be generated based upon $(1 - A1/A2)$, with A1 being an area between the amplitude of the obtained electrical signal and a nearest boundary of the range when the amplitude of the obtained electrical signal is outside of the range, and with A2 being an area occupied by the range.

In the event that A1 is greater than A2, the second plant specimen data may be given a zero value.

The health state of the group of plants within the growth environment may be assessed based upon an average of the plant data of the plurality of plant specimens.

Assessing a health state of the group of plants may comprise generating group growth index data based upon an average of the plant data of the plurality of plant specimens. The group growth index data may be indicative of the health state of the group of plants within the growing environment.

The group growth index data may indicate a stress level of the group of plants within the growth environment.

Generating the plant data may comprise generating individual growth index data of the respective plant specimen based upon the first plant specimen data and the second plant specimen data.

In other words, the plant data may comprise the individual growth index data.

The individual growth index data may indicate a stress level of the respective plant specimen.

The individual growth index data may be generated based upon (the first plant specimen data + the second plant specimen data)/2 + abs (the first plant specimen data - the second plant specimen data)/4, wherein abs() returns an abstract value.

The group growth index data and the individual growth index data may also be referred to as "first or group health status data" and "second or individual health status data", respectively.

The health state of the group of plants within the growth environment may be assessed based upon an average of the individual growth index data of the plurality of plant specimens.

Assessing a health state of the group of plants within the growth environment may comprise:

calculating an average of the first plant specimen data of the plurality of plant specimens;
calculating an average of the second plant specimen data of the plurality of plant specimens; and
calculating the group growth index data based upon the average of the first plant specimen data and the average of the second plant specimen data.

The group growth index data may be based upon (the average of the first plant specimen data + the average of the second plant specimen data)/2 + abs (the average of the first plant specimen data - the average of the second plant specimen data)/4, wherein abs() returns an abstract value.

It would be appreciated that each of the obtained electrical signal, the plant data, the first plant specimen data, the second plant specimen data, the individual growth index data and the group growth index data may comprise an array/series of data items, i.e., forming a 1-dimension matrix), with each data item corresponding to a time point or a unit time segment (e.g., 1 minute) within the first time period. In that case, the comparison and generation of data described above follow the basic rules of matrix calculations.

The method may further comprise sending an alert to a grower of the growth environment based upon the assessed health state of the group of plants.

The method may further comprise predicting a future yield of the group of plants based upon the assessed health state of the group of plants.

The method may further comprise generating a plant control signal based upon the assessed health state of the group of plants, wherein the plant control signal is configured to change the growth environment.

The obtained electrical signal may comprise electrical signal samples obtained from the respective plant specimen at a sequence of time points during the first time period.

The first time period may occur within a single day.

It would be understood that the second time period may occur temporally before the first time period.

The second time period may be longer than the first time period.

The second time period may comprises a plurality of sub-periods each corresponding to the first time period but taking place at least one day before the first time period.

With the expression "corresponding to the first time period", it is meant that each sub-period occurs at the same hours/minutes as the first time period, and has the same temporal length as the first time period.

It would be understood that the plurality of sub-periods span across a plurality of days before the first time period.

The historical electrical signal may comprise historical electrical signal samples obtained from the respective plant specimen at the sequence of time points during each of the sub-periods.

It would be understood that in the present disclosure, the term "time points" refer to the hours/minutes/seconds irrespective of the date, while the terms "time periods" and "sub-periods" take into account the date and can be distinguished from one another by the occurring dates.

The data indicative of the historical electrical signal may comprise a series of historical data corresponding to the sequence of time points respectively, and each historical data comprises data indicative of a weighted average and data indicative of a weighted standard deviation of amplitudes of the historical electrical signal samples that were obtained at a respective time point across the plurality of sub-periods.

The plurality of sub-periods may comprise a first sub-period and a second sub-period. The second sub-period may be temporally closer to the first time period than the first sub-period. The historical electrical signal samples obtained during the second sub-period may have a higher weight than the historical electrical signal samples obtained during the first sub-period.

The method may further comprise obtaining the historical electrical signal, and calculating the data indicative of the historical electrical signal.

Calculating the data indicative of the historical electrical signal may comprise: for each of the sequence of time points, calculating the weighted average and the weighted standard deviation of amplitudes of the historical electrical signal samples that were obtained at the respective time point across the plurality of sub-periods, so as to obtain a series of weighted averages corresponding to the sequence of time points respectively, and a series of weighted standard deviations corresponding to the sequence of time points respectively; smoothing the series of weighted averages, and smoothing the series of weighted standard deviations.

The series of historical data may comprises the series of smoothed weighted averages (i.e., data indicative of the weighted average) and the series of smoothed weighted standard deviations (i.e., data indicative of the weighted standard deviation). Smoothing the series of weighted averages (and/or weighted standard deviations) may comprise calculating a moving average of a subset of the series of weighted averages (and/or weighted standard deviations).

At each of the sequence of time points, the range of the amplitude of the historical electrical signal may have a lower boundary which is defined by the data indicative of the weighted average (e.g., the smoothed weighted average) minus the data indicative of the weighted standard deviation (e.g., the smoothed standard deviation) corresponding to the respective time point, and may have a higher boundary which is defined by the data indicative of the weighted average (e.g., the smoothed weighted average) plus the data indicative of the weighted standard deviation (e.g., the smoothed standard deviation) corresponding to the respective time point.

Comparing the obtained electrical signal with data indicative of the historical electrical signal may comprise: comparing the amplitude of the electrical signal sample obtained from the plant specimen at each of the sequence of time points during the first time period, with the range of the amplitude of the historical electrical signal corresponding to the respective time point.

The sequence of time points may be divided into one or more time segment each comprising more than one adjacent time points.

Generating the first plant specimen data may comprise, for each time segment:

counting a number of time point(s) at which the amplitude of the electrical signal sample obtained from the plant specimen stays within a range of the amplitude of the historical electrical signal; and
calculating the first plant specimen data for the respective time segment as the counted number of time point(s) divided by a total number of time points within the respective time segment.

Generating the second plant specimen data may comprise, for each time segment:

at each time point within the respective time segment:

determining, whether the amplitude of the electrical signal sample obtained from the plant specimen exceeds a range of the amplitude of the historical electrical signal, and if, but only if, the amplitude of the electrical signal sample exceeds the range, calculating a distance D1 between the amplitude of the electrical signal sample and a nearest boundary of the range; and
calculating a distance D2 between two boundaries of the range;

calculating A1 as a sum of D1 for all of the time point(s) at which the amplitude of the electrical signal sample exceeds the range;
calculating A2 as a sum of D2 for all of the time points within the respective time segment; and
calculating the second plant specimen data for the respective time segment as 1 - A1/A2.

The individual growth index data and/or the group growth index data may comprise an array of data element(s) corresponding to the at least one time segment, respectively.

The electrical signal and the historical electrical signal may be obtained (or sensed) by one or more capture devices comprising first and second electrodes.

The first electrode may be attached to a respective plant specimen, and the second electrode may be attached to a growth medium of the respective plant specimen or an alternative reference site (e.g., the stem or the root) of the respective plant specimen.

The first electrode and the second electrode may be attached to two different parts of a respective plant specimen, with the two different parts having different distances from a growth medium of the respective plant specimen.

There is also provided a computer readable storage medium carrying computer readable instructions arranged to cause a computer to carry out a method described herein.

There is also provided a system for assessing a health state of a group of plants within a growth environment, comprising: a processor; a computer readable storage medium carrying a computer program comprising computer readable instructions arranged to cause the processor to carry out a method as described herein; and one or more capture devices configured to sense the electrical signal and the historical electrical signal from the plurality of plant specimens within the growth environment.

The system may further comprises a plant control system configured to automatically change the growth environment based upon the plant control signal.

Where appropriate any of the optional features described above in relation to the method may be applied to the computer readable medium and system also described herein.

Brief Description of the Drawings

In order that the disclosure may be more fully understood, a number of embodiments of the disclosure will now be described, by way of example, with reference to the accompanying drawings, in which:

Figure 1 schematically illustrates plant health monitoring devices and a plant control/assessment device in use with plants within a growth environment;

Figure 2 is a schematic illustration of exemplary components that may be provided by each plant health monitoring device shown in Figure 1;

Figure 3 is a schematic illustration of exemplary components that may be provided by the plant control/assessment device shown in Figure 1;

Figure 4 is a flowchart showing processing steps carried out by the plant health monitoring devices and the plant control/assessment device to assess a health state of a group of plants within the growth environment;

Figure 5 shows exemplary time-domain waveforms of an electrical signal obtained from a plant specimen and data indicative of a historical electrical signal obtained from the same plant specimen;

Figure 6 is a flowchart showing processing steps for generating the data indicative of the historical electrical signal;

Figure 7 shows exemplary time-domain waveforms of an electrical signal obtained from a plant specimen, and Fit, Accuracy and individual growth index data of the same plant specimen;

Figure 8 shows an exemplary waveform of the GGI array and a heat-map;

Figure 9 illustrates a relationship between GGI metric values and real yields of tomato plants at 4 weeks later;

Figure 10 is a flowchart showing processing steps for predicting the future yield of a group of plants based upon GGI data;

Figure 11 shows exemplary time-domain waveforms of a GGI data array and predicted yields; and

Figure 12 shows an exemplary weekly plant health metric report provided to a grower of the growth environment 50.

In the figures, like parts are denoted by like reference numerals.

It will be appreciated that the drawings are for illustration purposes only and are not drawn to scale.

Detailed Description of the Preferred Embodiments

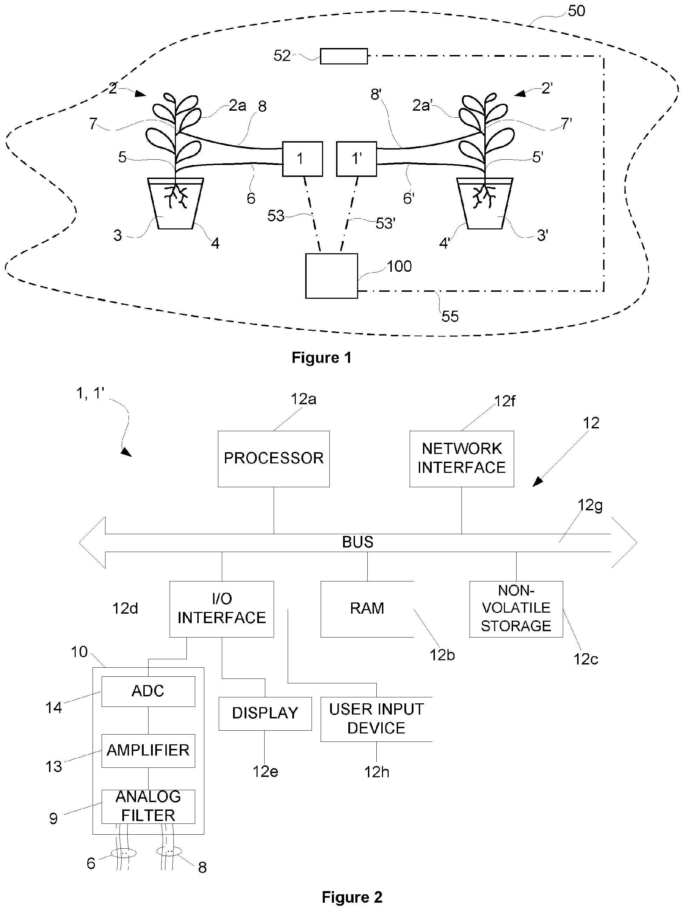

As shown in Figure 1, a plant health monitoring device 1 (hereinafter, monitor 1) is arranged to obtain electrical signals from a plant 2 and to process the obtained electrical signal so as to monitor the health of the plant 2. The plant 2 is grown in a growth medium 3 which is contained within a container 4. The growth medium 3 may be any suitable medium in which the plant 2 is able to grow. For example, the medium 3 may be soil, compost, water or the like.

A reference electrode 5 is attached to or inserted into a stem of the plant 2, and is connected, by a lead 6, to the monitor 1. A capture electrode 7 is attached to a petiole of a leaf 2a of the plant 2, and is connected, by a second lead 8, to the monitor 1. Alternatively, the reference electrode 5 may be inserted into, or otherwise attached to, a portion of root of the plant 2, or into the growth medium 3. Attaching the reference electrode 5 to the stem/root of the plant 2 allows signal uncertainties coming from the medium 3 to be removed. For example, it has been found that variations in the moisture level of the medium 3 result in variations in the electrical signal sensed at the reference electrode 5. Therefore, it is more preferable to attach the reference electrode 5 to the stem/root of the plant 2 than to insert it into the medium 3.

In an example where both the reference electrode 5 and the capture electrode 7 are attached to the plant 2, it is preferable to attach the reference electrode 5 to a part of the plant 2 which is closer to the growth medium 3, and to attach the capture electrode 7 to another part of the plant 2 which is further away from the growth medium 3.

It will be appreciated that the capture electrode 7 may comprise a plurality of capture electrodes which can be attached to a plurality of plants 2 or multiple different parts of a single plant 2. Similarly, the reference electrode 5 may comprise one or more reference electrodes. In an exemplary set-up, the monitor 1 is connected to a plurality of reference electrodes 5 and a plurality of capture electrodes 7, and may be able to obtain electrical signals from more than one plant or from different parts of the same plant, at the same time. The electrodes 5, 7 and the leads 6, 8 may be collectively referred to as a capture device of the plant 2 for sensing/obtaining an electrical signal from the plant 2.

Figure 1 further shows that the plant 2 is within a growth environment 50. The growth environment may be a controlled growth environment (such as a glasshouse, a greenhouse, a polytunnel, an indoor farm, or a vertical farm), or an open field. In general, a controlled growth environment provides more stable conditions for growing healthy crops, and can generate a yield ten times higher than a yield in an open field. Within the growth environment 50, there is another plant 2' and another monitor 1' arranged to obtain electrical signals from the plant 2' via leads 6', 8' and electrodes 5', 7'. The plant 2' is of the same type as the plant 2. It would be understood that components associated with the plant 2' are similar to those described in relation to the plant 2, and thus are omitted from description for brevity. While Figure 1 shows that there are only two plants 2, 2' within the growth environment 50, it would be understood that the growth environment 50 may accommodate a large group of plants and that the plants 2, 2' are merely a subset of the group.

A device 52 is configured to change the growth environment 50. In an example, the device 52 comprises one or more relays, which are configured to control a heater, a fan or a vent provided within the growth environment 50, a lighting device which illuminates the plants 2, 2', a water pump which irrigates the plants 2, 2', a pump which deliver a chemical substance (e.g., salt or fertiliser) to the growth mediums 3, 3', a pump which applies fungicide or insecticide to the plants 2, 2', a device which controls the humidity within the environment 50, and/or a device which delivers nutrition to the plants 2, 2'. Further or alternatively, the device 52 may comprise one or more smart devices, such as, a smart heater, a smart fan, a smart lighting device, a smart pump etc.

There is also provided a plant control/assessment device 100 (hereinafter, device 100) in communication with the monitors 1, 1' via communication links 53, 53', respectively. The device 100 is in communication with the device 52 via a communication link 55. The communication links 53, 53' and 55 may be wired or wireless (e.g., internet, Bluetooth etc.). In a preferred embodiment, the communication links 53, 53' and 55 are wireless, and the monitors 1, 1', the device 100 and the device 52 are within the same wireless communication network (e.g., a wireless local area network (WLAN)). While Figure 1 shows that the device 100 is located within the growth environment 50, it would be understood that the device 100 can be placed at any suitable place as long as it remains in communication with the monitors 1, 1' and the device 52.

The communication links 53, 53' may be direct or indirect links (via another device(s)) between the monitors 1, 1' and the device 100. The monitors 1, 1' obtain (or receive) electrical signals from the plants 2, 2', and the electrical signals are used to generate plant data indicative of a characteristic of the plants 2, 2', either by the monitors 1, 1' or another device(s) (not shown in Figure 1) connected to the monitors 1, 1'. As described in more detail below, the device 100 receives the plant data from the monitors 1, 1', and assesses a health state of a group of plants within the growth environment 50 based upon the plant data.

Based upon an assessment of the health state of the group of plants within the growth environment 50, the device 100 may be able to generate a plant control signal. The plant control signal can be used to automatically change an operation of the device 52 (via the communication link 55) without human intervention so as to change the growth environment 50. Therefore, the device 100 and the device 52 collectively provide a plant control system. By changing the operation of the device 52, it would be appreciated that an operational mode, a throughput and/or an output power of the device 52 may be increased or decreased, or the device 52 may be switched on or off. In the event that the device 52 is a relay, the plant control signal simply switches on or off the device 52. The device 100 may also be referred to as a central processing device which gathers and processes the data received from each of the monitors 1, 1'. It would be appreciated that the communication link 55 may be omitted and the device 52 may be controlled in a different way (e.g., not by the device 100).

The monitors 1, 1' allow either short-term or long-term monitoring of electrical signals obtained from the plants 2, 2'. The exemplary components of the monitor 1 are illustrated by Figure 2. The following description similarly applies to the monitor 1'. As shown in Figure 2, the monitor 1 comprises a data acquisition module 10 and a controller 12. The leads 6, 8 are connected to inputs of the data acquisition module 10. The data acquisition module 10 measures voltage potential differences present between each pair of the electrodes 5, 7 attached to the plant 2. Electrical signals may be recorded in mV level as a function of time and may be recorded at a rate of 240 Hz (i.e., 240 samples per second) by the data acquisition module 10. The recording frequency of the data acquisition module 10 may, for example, be any value between 1 Hz to 10 KHz.

In more detail, the data acquisition module 10 comprises an analog filter 9, an amplifier 13, and an analog-to-digital converter (ADC) 14. The analog filter 9 may be a low-pass filter. In an example, the analog filter 9 may be a DC-30Hz filter with a gentle 6dB/octave roll off. Such a filter is useful for minimal ringing so that the transient waveforms have minimal distortion in the time domain. The amplifier 13 may be an analog, non-switching, instrumentation amplifier and may provide an amplification factor between 0 and 100. In an example, the amplifier 13 provides an amplification factor of 4.

The ADC 14 may be of a Successive Approximation Register (SAR) design. In an example, the ADC 14 may be an 18-bit SAR ADC capable of processing 100K samples per second. In particular, the ADC 14 has a sample-and-hold input and may return a full 18-bit signed value over a +/- 2.048V range for each input. The ADC 14 may be implemented using ADS8777 ADC made by Texas Instruments.

The controller 12 comprises a processor 12a which is configured to read and execute instructions stored in a volatile memory 12b which takes the form of a random access memory. The volatile memory 12b stores instructions for execution by the processor 12a and data used by those instructions. For example, in use, the data acquired by the data acquisition module 10 may be stored in the volatile memory 12b.

The controller 12 further comprises non-volatile storage 12c which may be in the form of a hard disc drive or a solid state drive. The data acquired by the data acquisition module 10 may be stored on the non-volatile storage 12c. The controller 12 further comprises an I/O interface 12d to which are connected data capture and peripheral devices (e.g., display, input device, etc.) used in connection with the controller 12. As shown in Figure 2, the output of the data acquisition module 10 is connected to the I/O interface 12d. By virtue of these connections, potential differences sensed at the electrodes 5, 7 can be processed and converted to a digital signal by the data acquisition module 10 and subsequently processed by the processor 12a and stored in the non-volatile storage 12c. A display 12e is also connected to the I/O interface 102d. The display 12e displays a user interface which allows a user to input user defined rule set(s). This is described in more detail below. The display 12e may be provided locally to the monitor 1 (e.g. as a screen), or remotely from the monitor 1. For example, a display associated with a separate device (e.g. a mobile computing device or a desktop monitor) may be used as a display for the monitor 1. A user input device 12h is also connected to the I/O interface 12d. The user input device may be a mouse and/or a keyboard. Alternatively or additionally, a touchscreen may be associated with the display 12e to operate as the user input device 12h. A network interface 12f allows the controller 12 to be connected to an appropriate computer network so as to receive and transmit data from and to other computing devices. The processor 12a, volatile memory 12b, non-volatile storage 12c, I/O interface 12d, and network interface 12f, are connected together by a bus 12g.

The controller 12 may be connected to an external computer/server via the network interface 12f. In that case, the external computer/service may further process the digitalised signals obtained by the controller 12. In an example, the digitalised signals may be extracted and processed using a data processing software by the external computer/server. Further or alternatively, the controller 12 may be connected to a single board computer via the network interface 12f, such that the digitalised signals obtained by the controller are collected into the single board computer.

In general, due to natural and man-made terrestrial electromagnetic noise as well as the low-voltage level of the potential variations produced by the plant 2, a Faraday cage is typically used to surround the plant 2, the leads 6, 8 and the monitor 1 when electrical signals are obtained from the plant 2. The Faraday cage acts to shield the plant 2, the leads 6, 8 and the monitor 1 from external sources of electromagnetic radiation.

In order to allow for operating the monitor 1 outside of a Faraday cage, one or more of the following design implementations may be made to the monitor 1.

Electrostatic discharge (ESD) protection circuits may be provided to the inputs of the data acquisition module 10. The pre-ADC analog filter 9 may be designed for minimal overshoot and ringing so that the transient waveforms have minimal distortion in the time domain. As described above, in an example, the analog filter may be a DC-30Hz filter with a gentle 6dB/octave roll off. An ultra-low input bias current instrumentation amplifier may be selected for use as the amplifier 13. The analog filter 9, the amplifier 13, and the ADC 14 may be typically implemented on a printed circuit board (PCB). In that case, the PCB may be designed such that it includes multiple (e.g., at least two) ground planes connected to one another by a plurality of vias, thereby ensuring short electrical ground current return paths. Further, the ADC 14 may be designed such that the analog input signal to the ADC 14 is oversampled (i.e., sampled at a sampling rate significantly higher than the Nyquist rate), and the output signal of the ADC 14 is then processed through a digital filter and a decimator. Moreover, a digital notch filter, such as a 50Hz/100Hz or 60Hz/120Hz digital notch filter may be included to process the output signal of the ADC 14.

In addition to the above described design implementations to the monitor 1, the leads 6, 8 may be coaxial cables which are arranged to connect each of the electrodes 5, 7 to the monitor 1. The coaxial cables may be useful for shielding the leads 6, 8 from external sources of electromagnetic radiation.

For the ease of description, the coaxial cable connected to the reference electrode 5 is referred to as a "reference cable" and the coaxial cable connected to the capture electrode 7 is referred to as a "capture cable". A coaxial cable typically comprises an inner conductor surrounded by a tubular insulating layer, further surrounded by a tubular outer conducting shield. The inner conductors of the coaxial cables act as the leads 6, 8 and are used to transfer signals sensed by the electrodes 5, 7 to the monitor 1. The outer conducting shields of the coaxial cables act as EM shields to shield electromagnetic interference on the signals sensed by the electrodes 5, 7.

The coaxial cable has a capacitance between its inner conductor and its outer conducting shield. The capacitance of the coaxial cable may distort the signals sensed by the electrodes 5, 7. Thus, to compensate for the capacitance of the capture coaxial cable, a compensation circuit may be provided with the capture cable.

In more detail, the capture coaxial cable may be electrically connected to the capture electrode 7 via its inner conductor alone. The inner conductor of the capture cable provides an electrical signal sensed by the capture electrode 7 to the monitor 1. The compensation circuit includes a buffer circuit with an amplification factor of '1' for the capture cable. The buffer circuit receives a voltage signal from the inner conductor of the capture cable and outputs a voltage signal to drive the outer conducting shield of the same cable. In this way, the voltage difference between the inner conductor and the outer conducting shield of the capture coaxial cable is maintained at substantially 0 Volt by the buffer circuit. Accordingly, the capacitance of the capture cable does not charge or discharge based upon signal level fluctuations, and the distortion caused by the capacitance of the capture cable to the signals sensed by the electrodes 5, 7 may be reduced to a negligible level. The buffer circuit may, for example, take the form of a voltage follower. The voltage follower may be an op-amp circuit which has a negative feedback and a voltage gain of '1'.

The reference cable may be electrically connected to the reference electrode 5 via both of its inner conductor and outer conducting shield. That is, the inner core and the outer conducting shield of the reference cable may be electrically coupled together with a voltage difference of substantially 0 Volt. The outer conducting shield is driven with a voltage of 0 Volt (i.e. ground), to provide a low impedance path for channeling any interference to the ground. The inner conductor of the reference cable, which is also at a voltage of substantially 0 Volt, provides an electrical signal to the monitor 1. In this configuration, the reference cable does not require a compensation circuit to compensate for the capacitance of the cable.

Alternatively, the reference cable may be connected to the reference electrode 5 via its inner conductor alone, and accordingly may have its own compensation circuit as described above with reference to the capture cable. In either of these two configurations for the reference cable, the voltage difference between the inner core and the outer conducting shield of the reference cable is substantially zero. Accordingly, the capacitance of the reference cable does charge or discharge during signal acquisition, so its effect to the electrical signal sensed by the electrodes 5, 7 may be considered to be insignificant. Thus, transient distortions of the electrical signals obtained from the reference electrode 5 as a result of cable capacitance may be considered to be insignificant.

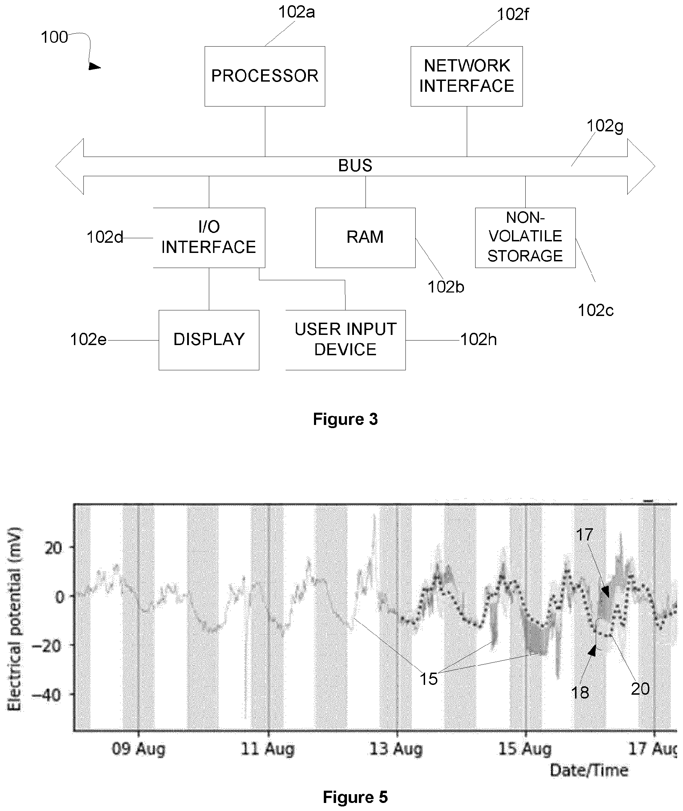

Figure 3 illustrates schematically the exemplary components of the device 100. As shown in Figure 3, the device 100 comprises a processor 102a, a volatile memory 102b, a non-volatile storage 102c, an I/O interface 102da, a display 102e, a user input device 102h, a network interface 102f and a bus 102g. These components of the device 100 are similar to the processor 12a, the volatile memory 12b, the non-volatile storage 12c, the I/O interface 12da, the display 12e, the user input device 12h, the network interface 12f and the bus 12g of the controller 12 of the monitor 1, respectively.

While Figure 1 shows that the monitors 1, 1' and the device 100 are separate devices, it would be appreciated that the functionality of the device 100 may be incorporated into one of the monitors 1, 1'.

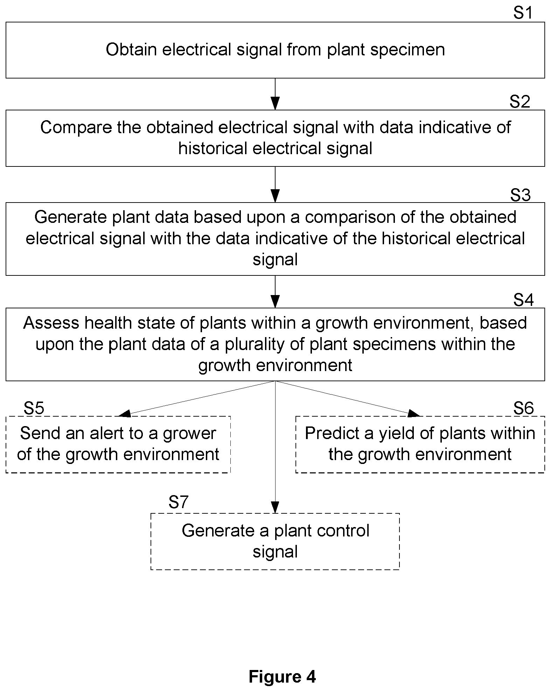

Figure 4 shows processing steps for assessing a health state of a group of plants within a growth environment (e.g., the growth environment 50). The processing steps may be carried out by the monitors 1, 1', the device 100, and/or an external computer/server which is connected to the monitors 1, 1' or the device 100 via the network interface 12f or 102f.

At step S1, an electrical signal is obtained from each of a plurality of plant specimen (e.g., the plants 2, 2') during a first time period.

The group of plants are generally of the same type (e.g., same genus, same species and/or same variety), and the plants 2, 2' are merely a subset of the group and act as the plant specimens of the group. The plant specimens may also be referred to as sentinel plants and are used to represent the group of plants within the growth environment.

The use of sentinel plants to monitor crop health is widely accepted in agriculture. Farmers employ plant "scouts" to visually look for crop stress on a regular basis. In the present disclosure, a subset of the group of plants are defined as sentinel plants because: (1) environmental conditions within a growth environment (e.g., a greenhouse) may be heterogeneous, and by sampling plants from several locations within the growth environment, it is possible to have plant readings from almost all the micro-climates of the growth environment; (2) adjacent plants generally behave similarly. Based upon the inventors' finding, inter-device variability is greater than intra-device variability; (3) taken together, it is possible to spot potential threats or abnormal environmental conditions by detecting changes in sentinel plants. Therefore, a health state of a group of plants within a growth environment can be confidently assessed by monitoring a lower number of sentinel plants.

While Figure 1 shows that there are only two plant specimens (i.e., plants 2, 2'), in reality four or more plant specimens scattered within the growth environment are typically monitored to obtain a reasonable representation of the group. In the event that the growth environment is a glasshouse compartment, sixteen plant specimens within an area of 1 hectare (which may contain 25,000 plants in total) are typically monitored. The most preferable number of plant specimens to monitor per glasshouse hectare may be 32 plant specimens arranged according to a layout as shown in Table 1. In general terms, the most preferable number of plant specimens depends on the glasshouse orientation, climate system vents and fans and the location of doors and passage ways.

Table 1: an exemplary layout of the plant specimens within the growth environment

| 4 plants | | 4 plants | | 4 plants |
|---|---|---|---|---|
| | 4 plants | | 4 plants | |
| 4 plants | | 4 plants | | 4 plants |

The electrical signal may be a time-domain waveform. An example of the obtained electrical signal is shown in Figure 5 as a signal 15. With reference to the setting of Figure 1, the electrical signal 15 represents the voltage potential difference between each pair of reference electrode 5 (or 5') and capture electrode 7 (or 7').

The electrical signal is typically in millivolts (mV), and may be sampled (e.g., by the data acquisition unit 10 of Figure 2) or re-sampled by the device 100 at 1/60Hz which is equivalent to 1 sample per minute. Each sample forms a basic data element of the electrical signal.

The first time period may be the whole day of a particular date (e.g., 0:00:00 to 23:59:59 of 13 August 2021). At the sampling rate of 1/60Hz, one day of data would include 1440 samples. Therefore, the electrical signal obtained from each plant specimen during a whole day may take the form of a one-dimensional data array $M_i$, which comprises 1440 data elements (i.e., $M_i[0]$, $M_j[1]$,... $M_i[1439]$). $M_i[0]$ is representative of a millivolt sample associated with the time point 0:00 (i.e., the unit time period 0:00:00 through 0:00:59) on 13 August 2021 while $M_i[1439]$ would be associated with the time point 23:59 (i.e., the unit time period 23:59:00 through 23:59:59) on 13 August 2021. In the data array $M_i$, 'i' represents the identity of a particular plant specimen, and may be one of 1, 2...'PS', with 'PS' being the total number of plant specimens being monitored. In other words, the data array $M_i$ includes electrical signal samples obtained from the particular plant specimen at a sequence of time points (e.g., 0:00, 0:01...23:58, and 23:59) during the first time period.

At step S2, for each plant specimen, the electrical signal obtained at step S1 is compared with data indicative of a historical electrical signal, and the historical electrical signal was obtained from the respective plant specimen during a second time period.

The data indicative of the historical electrical signal gives insights into what is considered as the plant normal state and, importantly, when the instantaneous recording of plant electrical signal deviates from it, and therefore, can be used to explore the health status of the respective plant specimen.

As shown in Figure 5, an exemplary data indicative of the historical electrical signal includes a dotted time-domain waveform 20 and an area 18 surrounding the dotted time-domain waveform 20. If the electrical signal 15 obtained from a plant specimen at step S1 falls inside the area 18 associated with the same plant specimen, then it is considered that the plant specimen is behaving as expected.

In general, the data indicative of the historical electrical signal provides the expected range of amplitude values for a respective plant specimen in the following day. As described below in more detail, this future projection is helpful to identify immediate deviations and is used to trigger an alerting system (notifications) to a grower of the growth environment.

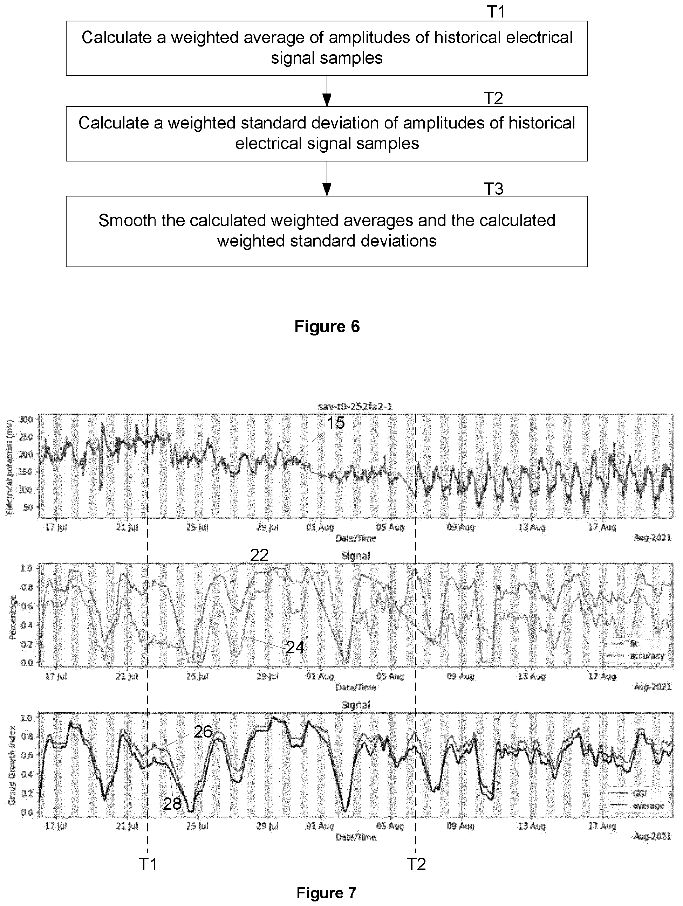

Figure 6 is a flowchart showing processing steps for generating the data indicative of the historical electrical signal (e.g., the time-domain waveform 20 and the area 18). The processing steps may be performed by a monitor (e.g., monitor 1 or 1') attached to the respective plant specimen, the device 100, or an external computer/server which is connected to the monitor or the device 100 via the network interface 12f or 102f.

The historical electrical signal may be obtained using the same device(s) (e.g., the monitor 1 or 1' via the electrodes 5, 7 and the leads 6, 8, and/or the device 100) and in the same manner as the electrical signal referred to at step S1. For example, the historical electrical signal may be sampled (e.g., by the data acquisition unit 10 of Figure 2) or re-sampled by the device 100 at 1/60Hz which is equivalent to 1 sample per minute, and each sample forms a basic data element of the historical electrical signal.

The second time period during which the historical electrical signal was obtained may comprise a plurality of sub-periods each corresponding to the first time period but taking place at least one day before the first time period. For example, if the first time period is the whole day of a particular date (e.g., 0:00:00 to 23:59:59 of 13 August 2021), the second time period may cover a plurality of sub-periods each spanning a whole day before the particular date (e.g., the plurality of sub-periods may be 0:00:00 to 23:59:59 of 9 August 2021, 10 August 2021, 11 August 2021 and 12 August 2021). The number of the sub-periods is at least two, e.g., the second time period may span a minimum of two days. In the following example, the number of the sub-periods is four and the second time period spans four days. It would be appreciated that it is possible to use more than 4 days (e.g., 30 days), and the number of days covered by the second time period depends upon various factors (e.g., plant characteristics and the growth environment etc.).

The historical electrical signal samples (hereinafter, "historical samples") obtained from a particular plant specimen i within the second time period (e.g., the preceding four days of the first time period) can be arranged in a one-dimensional data array $A_i$ in order of increasing time. The data array $A_i$ comprises 1440*D data elements, i.e., $A_i[O]$, $A_i[1]$, $A_i[2]$,... $A_i[1440*D-1]$. 'D' is the number of the sub-periods (e.g., four) included within the second time period, and 'i' indicates the identity of the plant specimen as described above.

In the event that D is equal to four, array elements $A_i[4320]$ through $A_i[5759]$ are representative of the most recent day's data while elements $A_i[O]$ through $A_i[1439]$ are representative of the least recent day's data. The most recent day and the least recent day are relative to the first time period (e.g., the current day). The preceding four days of the first time period are referred to "Current Day-1" (corresponding to data $A_i[4320]$ through $A_i[5759]$), "Current Day-2" (corresponding to data $A_i[2880]$ ... $A_i[4319]$), "Current Day-3" (corresponding to data $A_i[1440]$ ... $A_i[2879]$) and "Current Day-4" (corresponding to data $A_i[O]$ through $A_i[1439]$). Different sub-periods (e.g., days) within the second time period may have different levels of significance, and therefore may be associated with different weighting factors.

The weighting factors of the plurality of sub-periods within the second time period are arranged in a data array $W_i$ in order of increasing days. The data array $W_i$ comprises D data elements, i.e., $W_i[0]$, $W_i[1]$,...$W_i[D-1]$, and with i indicating the identity of the plant specimen. For example, in the event that D is equal to four, the weighting factor for "Current Day-4" is $W_i[0]$ while the weighting factor for "Current Day-1" is $W_i[3]$, The most recent day's data may be considered more significant and therefore has a higher weighting factor than other days' data. Therefore, by way of example only, $W_i[3]$ may be equal to 2.0 while each of $W_i[0]$, $W_i[1]$ and $W_i[2]$ may be equal to 1.0.

At step T1, for each of the time points (e.g., 0:00 to 23:59) included within the first time period, a weighted average of amplitudes of the historical samples that were obtained at the respective time point across the plurality of sub-periods (e.g., days) covered by the second time period is calculated. Consequently, a data array of the calculated weighted averages (denoted as $R_i$) corresponding to the sequence of time points included within the first time period is obtained. The data array $R_i$ comprises 1440 data elements, i.e., $R_i[0]$, $R_i[1]$, $R_i[2]$,... $R_i[1439]$, with 'i' indicating the identity of the plant specimen as described above.

More specifically, each data element $R_i[N]$ (with N=0, 1,...1439) of the data array $R_i$ is calculated according to Equation (1) below:

$$R_i[N] = \frac{\sum_{j=0}^{D-1}\left[W_i[j] * A_i[N + 1440 * j]\right]}{\sum_{j=0}^{D-1} W_i[j]}$$

$$(1)$$

In other words, each data element $R_i[N]$ in the array $R_i$ is calculated using a group of historical samples, and each historical sample in the group was obtained at the same absolute time point (e.g., 0:00) and has a temporal distance of one day from neighbouring sample(s) of the group.

At step T2, for each of the time points (e.g., 0:00 to 23:59) included within the first time period, a weighted standard deviation of amplitudes of the historical samples that were obtained at the respective time point across the plurality of days covered by the second time period is calculated. Consequently, a data array of the calculated weighted standard deviations (denoted as $S_i$) corresponding to the sequence of time points included within the first time period is obtained. The data array $S_i$ comprises 1440 data elements, i.e., $S_i[0]$, $S_i[1]$, $S_i[2]$,... $S_i[1439]$, with 'i' indicating the identity of the plant specimen as described above.

More specifically, each data element $S_i[N]$ (with N=0, 1,... 1439) is calculated according to Equation (2) below:

$$S_i[N] = \sqrt{\frac{\sum_{j=0}^{D-1}[W_i[j] * (A_i[N + 1440 * j] - R_i[N])^2]}{\frac{number\ of\ nonzero\ weights - 1}{number\ of\ nonzero\ weights}(\sum_{j=0}^{D-1} W_i[j])}}$$

$$(2)$$

Typically, the number of nonzero weights would be equal to the number of sub-periods covered by the second time period, i.e., 4 in this example.

Optionally, a multiplication factor (e.g., 1.2) may be applied to each data element of $S_i$, so as to obtain a modified

array $S_i'$. By using a multiplication factor greater than 1, the array $S_i'$ has greater values than $S_i$. This means that the height of the area 18 surrounding the dotted time-domain waveform 20 would be greater, and thus the expected ranges of amplitude values for the respective plant specimen would be wider.

At step T3, each of the data arrays $R_i$ and $S_i$ (or $S_i'$) is smoothed to dampen short-term variations in the respective data array. This process may also be referred to as applying a low pass filter to the data arrays in digital signal processing.

The data arrays $R_i$ and $S_i$ (or $S_i'$) may be smoothed by generating a new data point which is the average of a sliding window of 120 data elements. The results of the smoothing operation are stored in data arrays $RS_i$ and $SS_i$ (or $SS_i'$).

It is desired that the data arrays $RS_i$ and $SS_i$ (or $SS_i'$) contain the same number of data elements (e.g., 1440) as the data arrays $R_i$ and $S_i$ (or $S_i'$). Since there will be an insufficient number of 120 element sets to generate 1440 elements in $RS_i$ and $SS_i$ (or $SS_i'$), some data elements of $RS_i$ and $SS_i$ (or $SS_i'$) may be generated using fewer than 120 elements.

For example, $RS_i[0]$ to $RS_i[58]$ may be calculated as follows where the data window does not slide but rather increases in size from 61 elements initially to 119 elements:

$$RS_i[0] = (R_i[0] + R_i[1] + \cdots + R_i[60])/61$$

$$RS_i[1] = (R_i[0] + R_i[1] + \cdots + R_i[61])/62$$

$$RS_i[2] = (R_i[0] + R_i[1] + \cdots + R_i[62])/63$$

$$....$$

$$RS_i[58] = (R_i[0] + R_i[1] + \cdots + R_i[118])/119$$

Once the data window contains 120 elements, the average is generated as a moving average of the window as follows:

$$RS_i[59] = (R_i[0] + R_i[1] + \cdots + R_i[119])/120$$

$$RS_i[60] = (R_i[1] + R_i[2] + \cdots + R_i[120])/120$$

$$RS_i[61] = (R_i[2] + R_i[3] + \cdots + R_i[121])/120$$

$$....$$

$$RS_i[1379] = (R_i[1320] + R_i[1321] + \cdots + R_i[1439])/120$$

A moving average may also be referred to as "rolling average" or "running average". Given a series of numbers and a fixed subset size (e.g., 120), the first element of the moving average is obtained by taking the average of the initial fixed subset of the number series. Then the subset is modified by "shifting forward"; that is, excluding the first number of the series and including the next value in the series. In general, a moving average is useful to smooth out short-term fluctuations and highlight longer-term trends.

The data window then begins decreasing to generate the final 120 data elements:

$$RS_i[1380] = (R_i[1321] + R_i[1322] + \cdots + R_i[1439])/119$$

$$RS_i[1381] = (R_i[1322] + R_i[1323] + \cdots + R_i[1439])/118$$

$$RS_i[1382] = (R_i[1323] + R_i[1324] + \cdots + R_i[1439])/117$$

$$....$$

$$RS_i[1439] = (R_i[1380] + R_i[1381] + \cdots + R_i[1439])/60$$

The data array $SS_i$ (or $SS_i'$) may be generated in a way similarly to $RS_i$. Further, it would be appreciated that the multiplication factor (e.g., 1.2) may be applied to each data element of the array $SS_i$ instead of the array $S_i$, so as to obtain the modified array $SS_i'$.

Consequently, the smoothed data array $RS_i$ forms the dotted waveform 20, and the smoothed data array $SS_i$ (or $SS_i'$) indicates a height of the area 18 above and below the dotted waveform 20 for each time point within the first time period. It would be understood that the waveform 20, the area 18 and the electrical signal 15 have the same temporal resolution (i.e., 1 data sample per minute) according to the calculation above.

In the present disclosure, the data array $RS_i$ (i.e., the waveform 20) may also be referred to as a grow-wave, while

the data array $SS_i$ (or $SS_i'$) represents the standard deviation envelope of the grow-wave. The grow-wave can be interpreted as a prediction of the millivolt amplitude value that is expected to be obtained if the plant is measured on the current day, while the standard deviation envelope can be interpreted as limits of possible variations from the grow-wave.

Therefore, the time-domain waveform 20 and the area 18 collectively represent a range of an amplitude of the historical electrical signal, which is an expected range of the amplitude of the electrical signal obtained at step S1. More specially, at a particular time point N, the range of the amplitude of the historical electrical signal is between $RS_i[N]-SS_i[N]$ and $RS_i[N] + SS_i[N]$ (or between $RS_i[N] - SS_i'[N]$ and $RS_i[N] + SS_i'[N]$ if the multiplication factor is used).

Turning back to step S2 of Figure 4, comparing the electrical signal obtained at step S1 with the data indicative of the historical electrical signal involves comparing the amplitude of the electrical signal obtained at each time point of the first time period with the range of the amplitude of the historical electrical signal at the respective time point, i.e., comparing $M_i[N]$ with the range of between $RS_i[N]- SS_i[N]$ and $RS_i[N]+ SS_i[N]$ (or the range of between $RS_i[N]- SS_i'[N]$ and $RS_i[N]+ SS_i'[N]$) for each value of N (with N= 0, 1,2,...1439).

From the description above, it would be understood that the data elements in arrays $M_i$, $RS_i$, $SS_i$ and $SS_i'$ are aligned, such that $M_i[0]$, $RS_i[0]$, $SS_i[0]$ and $SS_i'[0]$ are all associated with the time point 0:00 while $M_i[1439]$, $RS_i[1439]$, $SS_i[1439]$ and $SS_i'[1439]$ are all associated with the time point 23:59.

At step S3, plant data is generated for each plant specimen, based upon a comparison of the obtained electrical signal with the data indicative of the historical electrical signal. The plant data is indicative of an extent of divergence between the obtained electrical signal and the historical electrical signal for the respective plant specimen.

At step S4, the health state of the group of plants within the growth environment during the first time period is assessed, based upon the plant data of the plurality of plant specimens.

Steps S5 to S7 are subsequent optional steps which may be performed, alone or in combination, based upon the GGI data array generated at step S4. Each of these steps is discussed in more detail below.

Referring again to the operation of Step S3, in an example, the plant data includes two error metrics - "Accuracy" and "Fit".

The error metric "Accuracy" (also referred to as first plant specimen data) indicates a percentage of time when the amplitude of the obtained electrical signal stays within the range of the amplitude of the historical electrical signal. The error metric "Fit" (also referred to as second plant specimen data) indicates an amount of deviation between the amplitude of the obtained electrical signal and the range of the amplitude of the historical electrical signal.

To calculate the error metrics "Accuracy" and "Fit", the sequence of time points (e.g., 0:00 to 23:59 which correspond to N=0, 1,....1439) within the first time period are divided into non-overlapping time segments (also called windows) each comprising more than one adjacent time points. In an example, each window is 5-minutes long, and thus the 1440 time points are divided into 288 windows. The error metrics "Accuracy" and "Fit" are generated for each window.

Within one window, a temporary counter is initially set to zero and the following logical argument is used in a loop for each time point within the window:

If $RS_i[N]- SS_i[N] < M_i[N] < RS_i[N]+ SS_i[N]$ is true, then the counter is incremented by 1.

For a 5-minute window (i.e., comprising 5 neighbouring time points), the counter could have a value ranging from 0, where no value $M_i[N]$ was within the grow-wave envelope, to 5 where all values $M_i[N]$ were between within the grow-wave envelope. The error metric "Accuracy" for each window is equal to the counter value of the respective window divided by 5 (i.e., the temporal length of the window).

Within one window, the area occupied by the grow-wave envelope (e.g., the area 18) may be calculated according to Equation (3) below:

$$A2 = \sum_{N=j}^{j+4} SS_i[N] * 2$$

(3)

As shown in Figure 5, the electrical signal obtained during the first time period at step S1 (i.e., the signal 15) does not always fall within the grow-wave envelope. The area 17 indicates an amount of deviation between the obtained electrical signal 15 and the grow-wave envelope.

To calculate the value of the area 17 (denoted as A1) for one window, A1 is initially set to zero and the following logical argument is used in a loop for each time point within the window:

If $RS_i[N] - SS_i[N] < M_i[N] < RS_i[N] + SS_i[N]$ is false, then A1 is incremented by $|M_i[N] - RS_i[N]|- SS_i[N]$.

In the logic argument above, $|M_i[N] - RS_i[N]|$ represents an absolute value of $M_i[N] - RS_i[N]$. The operation $|M_i[N] - RS_i[N]|- SS_i[N]$ therefore represents a distance between the obtained electrical signal and a nearest boundary of the grow-wave envelope.

The error metric "Fit" for each window is calculated as 1-A1/A2. If the calculated "Fit" value is negative (i.e., when the area 17 has a greater size than the area 18 for one window), the "Fit" value of that window is re-assigned a value of 0.0.

It would be understood that if the multiplication factor is used, $SS_i$ would be replaced by $SS_i'$ in the description above relating to the calculation of the "Accuracy" and "Fit" values.

The calculated error metrics "Accuracy" for all the windows within the first time period form a data array $ACCU_i$. The calculated error metrics "Fit" for all the windows within the first time period form a data array $FIT_i$. As described above, i is the identifier of the associated plant specimen. Each of the data arrays $ACCU_i$ and $FIT_i$ includes 1440WIN data elements, which correspond to the number of non-overlapping time segments (i.e., windows) included within the first time period. 'WIN' is the number of minutes included within each window. In the event that WIN is equal to 5 (as per the above example), each of the data arrays $ACCU_i$ and $FIT_i$ includes 288 data elements.

The plant data may also comprise individual growth index data array $GI_i$ of the respective plant specimen for each window within the first time period. The data array $GI_i$ includes the same number of data elements as the data array $ACCU_i$ or $FIT_i$. In an example, each data element $GI_i[L]$ (with L=0, 1, 2...287) of the data array $GI_i$ is defined according to Equation (4) below:

$$GI_i[L] = \frac{ACCU_i[L] + FIT_i[L]}{2} + \frac{|ACCU_i[L] - FIT_i[L]|}{4}$$

$$(4)$$

As per their definitions, the error metrics "Accuracy" and "Fit" are complementary, because "Accuracy" is specific for the time information and "Fit" is more sensitive to variations in amplitude. This means that these two error metrics, if taken individually, are generally insufficient to describe the plant health status. This is valid for individual plants but also for groups of plants.

$GI_i[L]$ is calculated by integrating both $ACCU_i[L]$ and $FIT_i[L]$ into a single value. It represents the health status of a plant specimen i at every time segment (e.g., 5-minute window) and varies between 0 (bad health status) and 1 (good health status). The value of $GI_i[L]$ is useful for comparing different plants within the same growth environment but could also serve as a starting point for an alerting feedback.

In Equation (4), $\frac{ACCU_i[L]+FIT_i[L]}{2}$ represents an average of $ACCU_i[L]$ and $FIT_i[L]$, while $\frac{|ACCU_i[L]-FIT_i[L]|}{4}$ represents a quarter of the difference between $ACCU_i[L]$ and $FIT_i[L]$. Therefore, $GI_i[L]$ sits at 75% between $ACCU_i[L]$ and $FIT_i[L]$. The reason for calculating $GI_i[L]$ in this way is two-fold:

Firstly, the "Fit" value typically gives a higher value than the "Accuracy" value. Therefore, it is desirable to make the individual growth index data stay between the "Accuracy" value and the "Fit" value.

Secondly, in extreme cases where the "Accuracy" and "Fit" have opposite values (e.g. one being close to 1 but the other being close to 0), it is not reasonable to excessively penalise the plant state. Therefore, it is desirable for the individual growth index data to sit closer to the higher value of "Accuracy" and "Fit".

One example of the extreme cases is called slow drift. Slow drift may cause the obtained electrical signal of a plant specimen to sit just outside the grow-wave envelope. This causes the "Accuracy" value to be close to 0, and the "Fit" value close to 1 because of the small amount of deviation in amplitudes. Depending on the height of the grow-wave envelope, this small deviation can be included or excluded (e.g., by using a multiplication factor that is slightly greater than 1). This means that small deviations do not necessarily indicate alarming plant health status. When this particular type of extreme cases happen, it is not desirable or reasonable to penalise the GI value. According to Equation (4), when "Fit" = 1, "Accuracy" = 0, the average of "Fit" and "Accuracy" = 0.5, but GI = 0.75 indicating that the plant specimen is in greater compliance with the grow-wave envelope and has better health than that indicated by the average of "Fit" and "Accuracy".

Another example of the extreme cases is called fast/strong deviation, which may be visible after watering events. In particular, after watering events, the obtained electrical signal may strongly deviate from the grow-wave envelope for a short period of time. Consequently, a sudden drop in "Fit" value may be measured while the "Accuracy" value stays close to 1. Considering that watering events are harmless, it is not reasonable to penalise the GI value. According to Equation (4), when "Fit" = 0, "Accuracy" = 1, the average of "Fit" and "Accuracy" = 0.5, but GI = 0.75 indicating that the plant specimen has better health than that indicated by the average of "Fit" and "Accuracy".

Figure 7 shows exemplary time-domain waveforms of $M_i$ (waveform 15), $ACCU_i$ (waveform 24), $FIT_i$ (waveform 22), $GI_i$ (waveform 26) and $\frac{ACCU_i+FIT_i}{2}$ (waveform 28). It would be understood that the temporal resolution of the waveforms 22, 24, 26 and 28 (e.g., 1 data sample per 5 minutes) is lower than that of the waveform 15 (e.g., 1 data sample per minute) according to the calculations above.

As shown in Figure 7, if $ACCU_i$ and $FIT_i$ are close (e.g., both being 1 or 0), then $GI_i$ would not deviate significantly from the average between $ACCU_i$ and $FIT_i$.

At time 'T1', $FIT_i$ is significantly greater than $ACCU_i$, and $GI_i$ deviates noticeably from the average of $ACCU_i$ and $FIT_i$.

At time 'T2', $ACCU_i$ is significantly greater than $FIT_i$, and $GI_i$ deviates noticeably from the average between $ACCU_i$ and $FIT_i$.

Therefore, Equation (4) is more accurate to assess a plant health status than simply using an average between $ACCU_i[L]$ and $FIT_i[L]$. It would be understood that Equation (4) is just an example, and may be modified such that, for example, $GI_i[L]$ sits at 65%-90% between $ACCU_i[L]$ and $FIT_i[L]$.

At step S4, the health state of the group of plants within the growth environment during the first time period is assessed, based upon the plant data of the plurality of plant specimens.

In an example, the health state of the group of plants is assessed based upon an average of the plant data of the plurality of plant specimens. During the assessment process, a group growth index data array GGI may be generated. GGI is indicative of the health state of the group of plants during the first time period. GGI is a grouped metric to describe the plurality of plant specimens (hence the group of plants) simultaneously.

The data array $GGI$ may have the same temporal resolution as the data array $GI_i$. That is, each data element of the data array GGI indicates a health state of the group of plants within a respective time segment (e.g., a 5-minute window) of the first time period. In other words, the data array $GGI$ may include the same number of data elements as the data array $GI_i$. In an example, each data element $GGI[L]$ (with L=0, 1, 2...287) of the data array $GGI$ is defined according to Equation (5) below:

$$GGI[L] = \frac{\sum_{i=1}^{PS} GI_i[L]}{PS}$$

(5)

Within the Equation (5), 'PS' is the total number of plant specimens being monitored in the growth environment. Because the data element $GI_i[L]$ is associated with a single plant specimen, $GGI[L]$ is defined as an average of the $GI_i[L]$ values of all plant specimens being monitored.

In general, the grow wave 20 (with its standard deviation envelope 18) shows a typical nycthemeral rhythm of a plant specimen in response to light. A stable rhythm occurs when the plant specimen is in stable growing conditions. Stress or high variability in the growing conditions causes the plant specimen's rhythm to deviate from a daily sinusoidal pattern. Therefore, the GI array $GI_i$ is a measure of the stress level or health status of a particular plant specimen i. According, being an average of the GI arrays $GI_i$ of multiple plant specimens, the GGI array is a measure of how a group of plants either maintain or deviate from a predicted rhythm, i.e., a measure of a general health state of the group.

Each data element of the GGI array is an index that spans from 0 (most, if not all, of the plants deviate from their expected behaviour; hence highly stressed or in a bad health state) to 1 (most, if not all, of the plants behave in accordance to their expected pattern; hence stress-free or in a good health state).

It would be understood that the first time period may be part of a whole day on a particular date (e.g., 5:00:00 to 23:00:00 of 13 August 2021). If so, then the second time period may comprise a plurality of discontinuous sub-periods each occurring between 5:00:00 and 23:00:00 in a number of days preceding the particular date (e.g., from 9 August 2021 to 12 August 2021).

Further, it would be understood that the processing steps of Figure 4 may be used for an *a posteriori* evaluation or a real-time evaluation of the group of plants within the growth environment 50. During the *a posteriori* evaluation, the first time period already occurred and step S1 essentially involves receiving the electrical signal from some storage device. During the real-time evaluation (i.e., online evaluation), steps S1 to S4 are performed to obtain/calculate each data element of the arrays $M_i$, $R_i$, $S_i$, $RS_i$, $SS_i$, $ACCU_i$, $FIT_i$, $GI_i$ and $GGI$ as the first time period progresses. For example, a new data element is added into the data array $M_i$ every minute as the current day progresses.

As described above, when generating the error metric arrays $ACCU_i$, $FIT_i$, the sequence of time points within the first time period are divided into non-overlapping time segments (e.g., each being a 5-minute window). It would be understood that the temporal length of the time segments may take a different value (e.g., 10 minutes, 20 minutes 30 minutes etc.). Generally speaking, shorter time segments would result in higher temporal resolution in $GI_i$ and $GGI$, which may be helpful for the identification of fast and shortlasting events but may also cause the final $GGI$ array to be excessively variable, thereby increasing the difficulty in identifying potential alerting events. On the other hand, long time segments may not allow certain events to be reflected in the final $GGI$ array. An intermediate resolution (e.g., each time segment being a 20-minute window) may be sufficiently sensitive to fast events while keeping the graph complexity low.

Optionally, at or after step S4, a standard deviation of the individual plant specimens' $GI_i$ may be calculated

according to Equation (6) below:

$$\sigma[L] = \sqrt{\frac{\sum_{i=1}^{PS}(GI_i[L] - GGI[L])^2}{PS}}$$

$$(6)$$

The data array $\sigma$ has the same temporal resolution as the data array *GGI*. That is, each data element of the array $\sigma$ corresponds to a time segment (e.g., a 5-minute window) of the first time period.

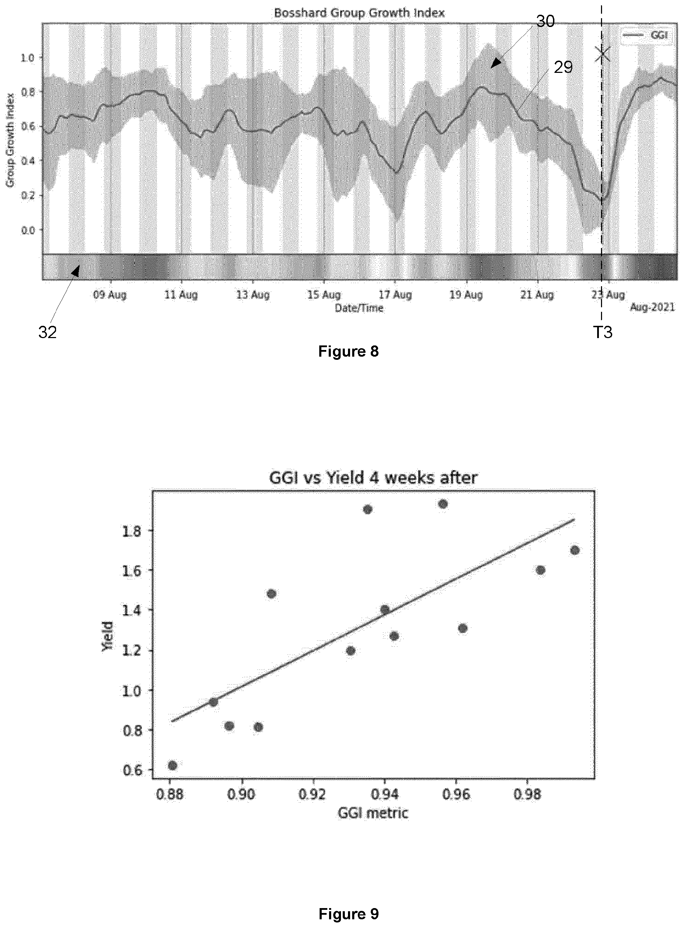

Figure 8 shows an exemplary waveform (i.e., waveform 29) of the GGI array. The waveform 29 shows the temporal evolution of the computed GGI values on 8 plant specimens. An area 30 surrounding the waveform 29 indicates the variability of the GGI array and is based upon the data array $\sigma$. In particular, a height of the area 30 above and below the waveform 29 at a particular time segment is equal to $\sigma[L]$, with L corresponding to the particular time segment.

Figure 8 also shows a colour-coded one-dimensional map 32 below the data/time axis. The map 32 may also be referred to as a heatmap. GGI values being close to 0 are represented with red colours in the heatmap 32 and GGI values being close to 1 are represented with green colours in the heatmap 32. Therefore, the heatmap 32 is a straightforward visual indication of the health status of the group of plants within the growth environment 50.

It can be seen in Figure 8 that at time 'T3' (around the 23rd of August), the GGI waveform 29 dropped to a very low value, and this drop was common for the majority of the plant specimens (because the height of the area 30 was short). Upon validation by the grower of the growth environment that was a greenhouse, a power outage lasted several hours on the 23rd of August. The power outage caused a disequilibrium in the environmental variables (temperature, $CO_2$, Humidity etc.) of the green house, thereby causing the plants to suffer from stress. Therefore, the GGI values were able to successfully capture this event and its impact on plant health.

The example described above is merely one approach for calculating the GGI values. In an alternative approach, the plant data generated at step S3 includes two error metric data arrays $ACCU_i$ and $FIT_i$ for each plant specimen. However, the individual growth index data array $GI_i$ of the respective plant specimen was not calculated at step S4. At step S4, average error metric arrays of all plant specimens, $ACCU_{ave}$ and $FIT_{ave}$, are firstly calculated. The data arrays $ACCU_{ave}$ and $FIT_{ave}$ may have the same temporal resolution as the data arrays $ACCU_i$ and $FIT_i$. That is, each data element of the data arrays $ACCU_{ave}$ and $FIT_{ave}$ is associated with a respective time segment (e.g., a 5-minute window) of the first time period. In an example, each data element (with L=0, 1, 2...287) of the data arrays $ACCU_{ave}$ and $FIT_{ave}$ is defined according to Equation (7) below:

$$ACCU_{ave}[L] = \frac{\sum_{i=1}^{PS} ACCU_i[L]}{PS}$$

$$FIT_{ave}[L] = \frac{\sum_{i=1}^{PS} FIT_i[L]}{PS}$$

$$(7)$$

Subsequently, the group growth index data array GGI is generated at step S4 based upon the average error metric data arrays $ACCU_{ave}$ and $FIT_{ave}$, according to Equation (8) below:

$$GGI[L] = \frac{ACCU_{ave}[L] + FIT_{ave}[L]}{2} + \frac{|ACCU_{ave}[L] - FIT_{ave}[L]|}{4}$$

$$(8)$$

It would be understood that both approaches would result in the same GGI data array.

With further reference to Figure 4, steps S5 to S7 are optional steps which may be performed, alone or in combination, based upon the GGI data array generated at step S4.

At step S5, an alert is automatically generated and sent to the grower of the growth environment 50 based upon the GGI data array. In an example, the alert is triggered when the following conditions are met: (i) data element(s) of the GGI data array drop below a critical threshold of 0.4; (ii) 50% or more of the plant specimens have a GI value that is below the GGI value during the same time segment(s) and (iii) an alerting period during which conditions (i) and (ii) are met lasts for at least 10 minutes. Condition (ii) is used to compensate for variability of individual plant responses to abiotic or biotic stress.

The conditions collectively define an alerting ruleset. It would be appreciated that the alerting ruleset may be suitably modified based upon characteristics of the growth environment and the group of plants.

The alert may be sent to an email or an email group provided by the grower. To avoid sending repetitive alerts on the same alerting condition, the alerting ruleset may be defined such that once an alert is sent, a further alert would not be sent within 120 minutes after the end of the alerting period. In general, the alert notifies the grower of undesirable events occurring within the growth environment and enables the grower to manually intervene so as to steer the development of plants.

At step S6, a future yield of the group of plants is predicted based upon the GGI data array. Yield predictions may cover the future one to eight weeks from the first time period.

The present disclosure has found that electrophysiological measurements of electrical signals of plants indicate a strong and regular diurnal rhythm, and that variations of the diurnal rhythm, both from a normal rhythm and variations in rhythm between plants, can be used to indicate stress in the plants. Variations of the diurnal rhythm occur as plants grow and develop, when climate conditions change, when irrigation or nutrigation events occur, when the plant is exposed to diseases or pests, and when human interventions (such as but not limited to, pruning, deleafing or applying an agrochemical) occur. Plants respond to all of these occurrences with differing intensities. The GGI data array therefore represents the stress response of the group of plants within the growth environment 50 and accordingly is a plant health measure of the group of plants.

Stress in plants often has a detrimental effect on yield. In particular, strong or long-duration variations of the diurnal rhythm suggest that the plant is diverting energy that would be used to grow and reproduce to other activities such as defence or healing. The present disclosure has found that the periods of strong variations of the diurnal rhythm correlate to reduced yields, while periods of compliance with the diurnal rhythm correlate to high yields in the coming weeks. Therefore, the GGI values of plants are correlated with future yield of the plants in the coming weeks. More specially, low GGI values mean that the future yield would just be a percentage of an ideal yield that plants could achieve.

Growers typically choose plant varieties that are expected to grow well in their specific growing operations. A key factor in choosing any plant variety is the expected yield which is often expressed as the maximum theoretical yield over the crop cycle. The maximum theoretical yield is never achieved because growers cannot maintain perfect plant health over the entire crop cycle. The actual yield is not known when the plants are planted or during the growth of the plants, and will vary depending on the growing conditions. Disrupted plant rhythms, poor water or nutrient conditions, poor climate conditions, pests and diseases all contribute to lower yields.

Figure 9 illustrates a relationship between GGI metric values and real yields of tomato plants at 4 weeks later than the date of the GGI metric values. The yield data (Y-axis) represents the weight of tomatoes (in kg) collected per square-meter per week. The GGI metric (X axis) is calculated based upon the GGI data array (1 data element per 5-min window) but re-scaled to the same time scale as the yield data (i.e., 1 data element per week). In particular, each data element of the GGI metric is equal to the number of data elements within the GGI array that is above the alerting threshold set out in condition (i) above (e.g., 0.4) divided by the total number of data elements of the GGI array within a week. Figure 9 shows that the yield data and the GGI metric data appear to have a linear relationship. In particular, high GGI metric values indicate stability/balance in the plants (reduced number of alerts) and correlate with an increased yield at 4 weeks in the future. When an increasing number of alerts are detected, the GGI metric decreases and this also correlates with a decreased yield at 4 weeks in the future. The GGI values of plants therefore provide reliable information for predicting future yield of the plants.

The yield prediction method provided by step S6 is purely based upon electrophysiological information obtained from the plants themselves, and requires no input related to plant genetics, climate conditions, growing operation design, nutrient or water supply. Further, the yield prediction method provided by the present disclosure works on any type of plant (e.g., ever-bearing, multi harvest and single harvest crops), and any growth environment.

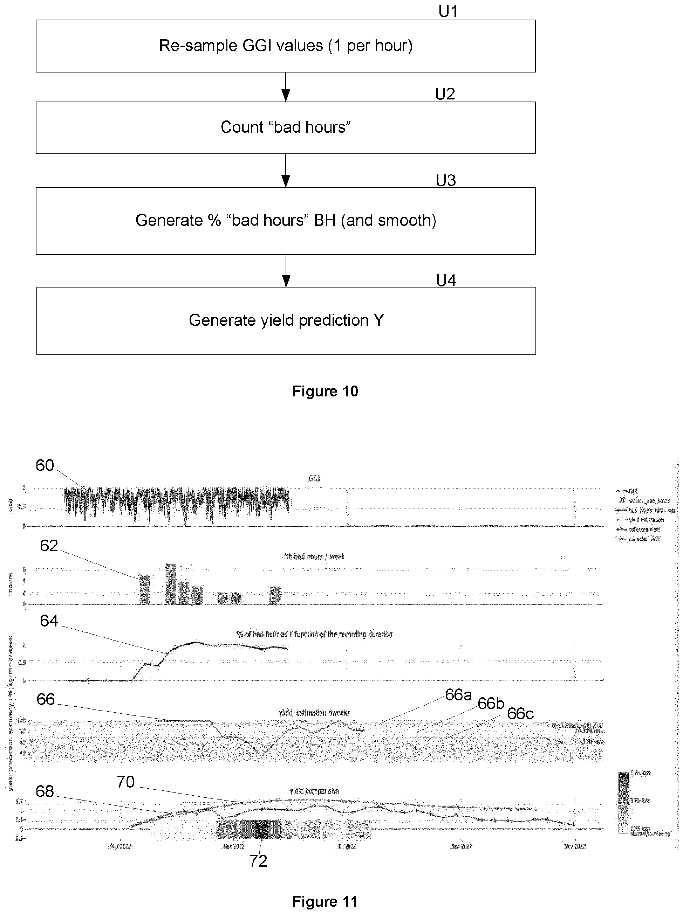

Predicting the future yield of the group of plants based upon the GGI data array may comprise the following processing steps, with reference to Figure 10.

In a first step U1, data elements of the GGI data are re-sampled to provide 1 data point for every hour. Such re-sampling may performed by taking a simple average of each of the (e.g. 12) GGI data items relating to a 1-hour period.

In a second step U2, the re-sampled data elements of the GGI data array that have values below a critical threshold (e.g., 0.235) are identified. The threshold value may be selected based on the particular type of crop, for example based on historical yield and GGI data. The number of identified hours may be referred to as a number of "bad hours".

In a third step U3 the number of bad hours may be converted to a percentage of hours in particular time period (e.g. a week, i.e. 168 hours) that have values below the critical threshold. The percentage value may be smoothed over a longer time period (e.g. a rolling two-week average).

In a fourth step U4, a predicted yield Y of the group of plants is generated based upon the percentage of "bad hours" BH in a given week w.

The yield may be predicted up to six weeks in advance for ever-bearing crops or at the end of the crop cycle for single bearing crops. The predicted yield may be a percentage of a maximum theoretical yield (when this number is known) or as a factor that can be applied to a typical yield that the grower achieves or has achieved in the previous weeks. The predicted yield may thus be calculated by applying a time-shift amount ts to the smoothed "bad hours" percentage BH from a given week w.

For example, for a tomato crop, a yield prediction for four or six weeks in the future may be based on current "bad hours" data. Alternatively, for cucumbers, the time-shift may be only two weeks. Different time shift values may be determined empirically based on historical yield and GGI data.

The bad hours percentage data BH may then be converted into a future yield estimation value Y according to Equation (9) below:

$$Y[w + ts] = 100 - 20 * BH[w]$$

(9)

The scaling factor (20) allows a convenient numerical range of values of Y, but other values could be used as required.

Figure 11 shows time-domain waveforms generated during the data processing for predicting the plant yield of a tomato crop being monitored according to the above described method. The waveform 60 is a GGI data array showing data generated over a period over around 20 weeks. The GGI data is re-sampled (e.g. by averaging) to provide a single data-point that represents each hour. The column chart 62 represents the temporal lengths (in hours) of GGI data elements below a threshold value. In the illustrated example the threshold value used was 0.235. The waveform 64 is a percentage bad hours BH of the temporal length 62, smoothed over two weeks. The waveform 66 represents the predicted yield in 6 weeks' time. When the predicted yield is between 90% and 100% (band 66a), it means a normal/increasing yield. When the predicted yield is between 70% and 90% (band 66b), it means that a 10-30% loss of the maximum/typical yield may be expected. When the predicted yield is below 70% (66c), it means that more than 30% loss of the maximum/typical yield may be expected.

The waveform 68 represents the real, collected, yield of the plants (e.g., measured by the weight of fruits in kg). The waveform 70 represents the nominal or expected yield of the plants (e.g., measured by the weight of fruits in kg).

With reference to Figure 11, it can be seen that many bad hours occurred during March and April 2022, and accordingly the predicted-yield waveform 66 shows that a reduced yield would be expected in May. This was confirmed by the real-yield waveform 68, which shows that 50% loss of maximum/typical yield occurred in mid-May. The chart 72 shows a heat map of yield loss, with the darkest region corresponding to 50% yield loss.

Referring again to the processing of Figure 4, at step S7, a plant control signal may be generated based upon the GGI data array. The plant control signal is configured to change the growth environment 50, by for example automatically changing an operation of the device 52.

In an example, the generation of the plant control signal may be triggered by the same conditions as defined in the alerting ruleset at step S5. The stressor that causes the undesirable events occurring within the growth environment may be identified using machine learning models as described in the Applicant's earlier patent applications (GB 2582547A and US2020/0302338-A1) which are incorporated herein by reference. The stressor may include one or more of: water deficit, insect infestation, $CO_2$ deficit, excess/insufficient nutrients, temperature stress, salt stress etc. Once the type of the stressor is identified, the plant control signal may be used to directly counter the stressor and to alter the growth environment by changing an operation of the device 52.

It is known that conditions (such as light, temperature, soil moisture etc.) of the growth environment 50 affect the health, crop yields and quality of plants. Therefore, by generating the plant control signal to control the growth environment 50, the growth environment 50 can be maintained with stable/ideal growing conditions to promote healthy crops, thereby leading to healthier plants and much higher crop yields and quality.

It would be understood that the type of the stressor identified may also be sent to the grower of the growth environment separately from or together with the alert sent at step S5 described above with reference to Figure 4.

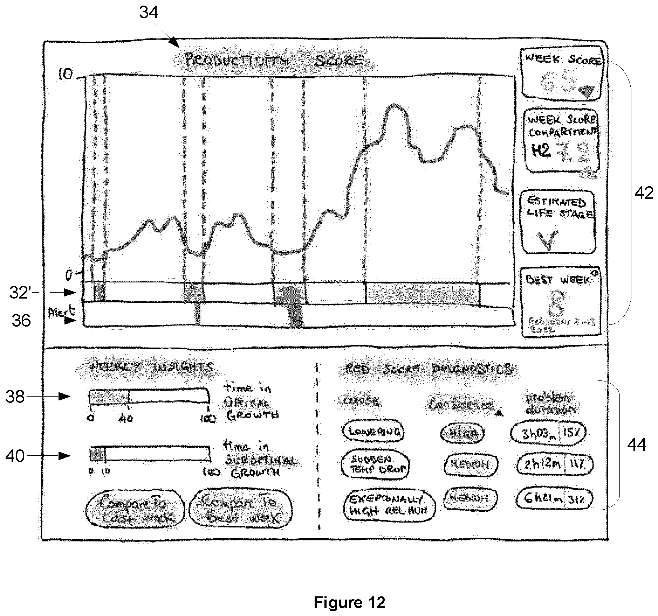

In addition, statistics data derived from the GGI data array as well as the identified stressors may also be sent to the grower, e.g., on a weekly basis. Figure 12 shows an exemplary weekly plant health metric report provided to the grower of the growth environment 50. The report includes a time-domain waveform of a productivity score 34 of the plants for a particular week. The productivity score of the plants may be based upon the GGI values but rescaled from 0 to 10 (i.e., x10). The report also includes a colour-coded one-dimensional heatmap 32' below the time axis of the productivity score waveform, and an alert map 36 indicating when alerts were sent to the grower. The heatmap 32' is similar to the heatmap 32 as shown in Figure 8. The report also includes weekly-based statistics data 42, such as a week score of the plants which for example is a mathematical average of the productivity scores within the particular week. The report also indicates a percentage of good performance period 38 and a percentage of poor performance period 40 of the plants within the particular week. The percentages 38, 40 are calculated based upon the heatmap 32'. The report further includes a section 44 which lists the identified types of stressors which caused the alerts to be sent. It would be appreciated that the layout and content of the report may be suitably modified.

It would also be understood that the terms "first" and "second" are simply used in the present disclosure to label the relevant elements ("time period", "data" etc.) for the ease of description, and do not imply any limitations to the sequence or total number of the relevant elements.

The terms "having", "containing", "including", "comprising" and the like are open and the terms indicate the presence of stated structures, elements or features but not preclude the presence of additional elements or features. The articles "a", "an" and "the" are intended to include the plural as well as the singular, unless the context clearly indicates otherwise.

Although the disclosure has been described in terms of preferred embodiments as set forth above, it should be understood that these embodiments are illustrative only and that the claims are not limited to those embodiments. Those skilled in the art will be able to make modifications and alternatives in view of the disclosure which are contemplated as falling within the scope of the appended claims. Each feature disclosed or illustrated in the present specification may be incorporated in the disclosure, whether alone or in any appropriate combination with any other feature disclosed or illustrated herein.

**Claims**

1. A method of assessing a health state of a group of plants within a growth environment, comprising:

    for each of a plurality of plant specimens within the growth environment, the plurality of plant specimens being a subset of the group of plants:

        obtaining an electrical signal from a respective plant specimen during a first time period;
        comparing the obtained electrical signal with data indicative of a historical electrical signal, with the historical electrical signal obtained from the respective plant specimen during a second time period; and
        generating plant data based upon a comparison of the obtained electrical signal with the data indicative of the historical electrical signal, wherein the plant data is indicative of an extent of divergence between the obtained electrical signal and the historical electrical signal for the respective plant specimen; and

    assessing the health state of the group of plants within the growth environment during the first time period, based upon the plant data of the plurality of plant specimens.

2. The method of claim 1, wherein the data indicative of the historical electrical signal represents a range of an amplitude of the historical electrical signal, and wherein optionally: comparing the obtained electrical signal with data indicative of the historical electrical signal comprises comparing an amplitude of the obtained electrical signal with the range of the amplitude of the historical electrical signal.

3. The method of claim 2, wherein generating the plant data comprises generating first plant specimen data indicating a percentage of time when an amplitude of the obtained electrical signal stays within the range, and generating second plant specimen data indicating an amount of deviation between the amplitude of the obtained electrical signal and the range.

4. The method of any preceding claim, wherein assessing a health state of the group of plants comprises generating group growth index data based upon an average of the plant data of the plurality of plant specimens, wherein the group growth index data is indicative of the health state of the group of plants within the growing environment.

5. The method of claim 3 or 4 as dependent from claim 3, wherein generating the plant data comprises generating individual growth index data of the respective plant specimen based upon the first plant specimen data and the second plant specimen data, and wherein optionally: the health state of the group of plants within the growth environment is assessed based upon an average of the individual growth index data of the plurality of plant specimens.

6. The method of claim 4 as dependent from claim 3, wherein assessing a health state of the group of plants within the growth environment comprises:

    calculating an average of the first plant specimen data of the plurality of plant specimens;
    calculating an average of the second plant specimen data of the plurality of plant specimens; and
    calculating the group growth index data based upon the average of the first plant specimen data and the average of the second plant specimen data.

7. The method of any preceding claim, further comprising:

sending an alert to a grower of the growth environment based upon the assessed health state of the group of plants; and/or
generating a plant control signal based upon the assessed health state of the group of plants, wherein the plant control signal is configured to change the growth environment.

8. The method of any preceding claim, further comprising: predicting a future yield of the group of plants based upon the assessed health state of the group of plants.

9. The method of any preceding claim, wherein the second time period comprises a plurality of sub-periods each corresponding to the first time period but taking place at least one day before the first time period.

10. The method of claim 9, wherein the obtained electrical signal comprises electrical signal samples obtained from the respective plant specimen at a sequence of time points during the first time period, and wherein the historical electrical signal comprises historical electrical signal samples obtained from the respective plant specimen at the sequence of time points during each of the sub-periods.

11. The method of claim 10, wherein the data indicative of the historical electrical signal comprises a series of historical data corresponding to the sequence of time points respectively, and each historical data comprises data indicative of a weighted average and data indicative of a weighted standard deviation of amplitudes of the historical electrical signal samples that were obtained at a respective time point across the plurality of sub-periods, wherein optionally:
the plurality of sub-periods comprises a first sub-period and a second sub-period, with the second sub-period being temporally closer to the first time period than the first sub-period, and wherein the historical electrical signal samples obtained during the second sub-period have a higher weight than the historical electrical signal samples obtained during the first sub-period.

12. The method of any preceding claim, wherein the obtained electrical signal comprises electrical signal samples obtained from the respective plant specimen at a sequence of time points during the first time period, and wherein the sequence of time points is divided into one or more time segment each comprising more than one adjacent time points.

13. The method of claim 12 as dependent from claim 3, wherein generating the second plant specimen data comprises, for each time segment:

at each time point within the respective time segment:

determining, whether the amplitude of the electrical signal sample obtained from the plant specimen exceeds a range of the amplitude of the historical electrical signal, and if, but only if, the amplitude of the electrical signal sample exceeds the range, calculating a distance D1 between the amplitude of the electrical signal sample and a nearest boundary of the range; and
calculating a distance D2 between two boundaries of the range;

calculating A1 as a sum of D1 for all of the time point(s) at which the amplitude of the electrical signal sample exceeds the range;
calculating A2 as a sum of D2 for all of the time points within the respective time segment; and
calculating the second plant specimen data for the respective time segment as 1 - A1/A2;
and/or
wherein generating the first plant specimen data comprises, for each time segment:

counting a number of time point(s) at which the amplitude of the electrical signal sample obtained from the plant specimen stays within a range of the amplitude of the historical electrical signal; and
calculating the first plant specimen data for the respective time segment as the counted number of time point(s) divided by a total number of time points within the respective time segment.

14. A computer readable storage medium carrying computer readable instructions arranged to cause a computer to carry out a method according to any preceding claim.

15. A system for assessing a health state of a group of plants within a growth environment, comprising:

a processor;
a computer readable storage medium carrying a computer program comprising computer readable instructions arranged to cause the processor to carry out a method according to any one of claims 1 to 13; and
one or more capture devices configured to sense the electrical signal and the historical electrical signal from the plurality of plant specimens within the growth environment;
wherein optionally: the system further comprises a plant control system configured to automatically change the growth environment based upon the plant control signal.

**Figure 1**

**Figure 2**

100

102a
PROCESSOR

102f
NETWORK
INTERFACE

102g

BUS

102d
I/O
INTERFACE

RAM

NON-
VOLATILE
STORAGE

102b

102c

102e
DISPLAY

USER INPUT
DEVICE
102h

**Figure 3**

**Figure 5**

S1

Obtain electrical signal from plant specimen

S2

Compare the obtained electrical signal with data indicative of historical electrical signal

S3

Generate plant data based upon a comparison of the obtained electrical signal with the data indicative of the historical electrical signal

S4

Assess health state of plants within a growth environment, based upon the plant data of a plurality of plant specimens within the growth environment

S5

Send an alert to a grower of the growth environment

S6

Predict a yield of plants within the growth environment

S7

Generate a plant control signal

**Figure 4**

| | T1 |
|---|---|
| Calculate a weighted average of amplitudes of historical electrical signal samples | |

| | T2 |
|---|---|
| Calculate a weighted standard deviation of amplitudes of historical electrical signal samples | |

| | T3 |
|---|---|
| Smooth the calculated weighted averages and the calculated weighted standard deviations | |

**Figure 6**

**Figure 7**

Figure 8

Figure 9

**Figure 10**

**Figure 11**

Figure 12

Europäisches Patentamt

European Patent Office

Office européen des brevets

# EUROPEAN SEARCH REPORT

**Application Number**

EP 24 19 3702

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2018/267006 A1 (WALLBRIDGE NIGEL CHRISTOPHER [CH] ET AL) 20 September 2018 (2018-09-20) * paragraphs [0095] - [0217]; figures 1, 4, 8 * | 1-15 | INV. G01N33/00 A01G9/26 |
| Y | US 2020/184153 A1 (BONGARTZ TIMO [DE] ET AL) 11 June 2020 (2020-06-11) * paragraphs [0494], [0495], [0669] * | 1-15 | |

**TECHNICAL FIELDS SEARCHED (IPC)**

G01N
A01G

The present search report has been drawn up for all claims

| Place of search | Date of completion of the search | Examiner |
|---|---|---|
| The Hague | 28 November 2024 | Wilhelm-Shalganov, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 3702

This annex lists the patent family members relating to the patent documents cited in  the above-mentioned European search report.
The members are as contained in the European Patent Office EDP file on
The European Patent Office is in no way liable for these particulars which are merely given for the purpose of information.

28-11-2024

| Patent document cited in search report | | Publication date | Patent family member(s) | | Publication date |
|---|---|---|---|---|---|
| US 2018267006 | A1 | 20-09-2018 | GB | 2541468 A | 22-02-2017 |
| | | | US | 2018267006 A1 | 20-09-2018 |
| | | | WO | 2017032750 A1 | 02-03-2017 |
| US 2020184153 | A1 | 11-06-2020 | NONE | | |

EPO FORM P0459

For more details about this annex : see Official Journal of the European Patent Office, No. 12/82